# EUROPÄISCHE PATENTSCHRIFT

(11) **EP 2 094 627 B1**
(45) Veröffentlichungstag und Bekanntmachung des Hinweises auf die Patenterteilung: **09.02.2011**
(21) Anmeldenummer: 07822801.2
(22) Anmeldetag: 22.11.2007
(51) Int. Cl.: C07C 2/76, C01C 1/04, C07C 15/04

(54) **INTEGRIERTES VERFAHREN ZUR HERSTELLUNG VON BENZOL UND AMMONIAK AUSGEHEND VON ALIPHATISCHEN KOHLENWASSERSTOFFEN UND STICKSTOFF**
INTEGRATED METHOD FOR PRODUCING BENZENE AND AMMONIA FROM ALIPHATIC HYDROCARBONS AND NITROGEN
PROCEDE INTEGRE POUR PRODUIRE DU BENZOLE ET DE L'AMMONIAC A PARTIR D'HYDROCARBURES ALIPHATIQUES ET D'AZOTE

(30) Priorität: 24.11.2006 EP 06124710
(43) Veröffentlichungstag der Anmeldung: 02.09.2009
(73) Patentinhaber: BASF SE, 67056 Ludwigshafen (DE)
(72) Erfinder: MÜLLER, Ulrich, 67435 Neustadt (DE); FREIBERGER, Harald, 67125 Dannstadt-Schauernheim (DE)
(74) Vertreter: Ziegler, Verena
(86) Internationale Anmeldenummer: PCT/EP2007/062668
(87) Internationale Veröffentlichungsnummer: WO 2008/062028

(56) Entgegenhaltungen:
- US-A- 4 689 208
- US-A- 4 727 206
- US-A1- 2002 072 642
- LIU C-J ET AL: "Comparative investigations on plasma catalytic methane conversion to higher hydrocarbons over zeolites" APPLIED CATALYSIS A: GENERAL, ELSEVIER SCIENCE, AMSTERDAM, NL, Bd. 178, Nr. 1, 8. März 1999 (1999-03-08), Seiten 17-27, XP004271663 ISSN: 0926-860X
- DATABASE WPI Week 199050 Derwent Publications Ltd., London, GB; AN 1990-371229 XP002479022 & JP 02 267116 A (AGENCY OF IND SCI & TECHNOLOGY) 31. Oktober 1990 (1990-10-31)

## Beschreibung

Die vorliegende Erfindung betrifft ein Verfahren zur integrierten Herstellung von Aromaten und Ammoniak aus einem C₁-bis C₆-Aliphaten und N₂ enthaltenden Gasstrom.

Aromatische Kohlenwasserstoffe wie Benzol, Toluol und Naphthalin stellen bedeutende Zwischenprodukte in der chemischen Industrie dar, deren Bedarf nach wie vor steigt. Sie werden in der Regel durch katalytische Reformierung aus Naphtha gewonnen, das seinerseits aus Erdöl erhalten wird. Neuere Untersuchungen zeigen, dass die weltweiten Erdölvorräte schneller erschöpft sein werden als die Erdgasvorräte. Die Herstellung von Benzol aus Erdgas als alternativem Rohstoff stellt deshalb eine interessante Alternative zu herkömmlichen Verfahren dar. Die Hauptkomponente von Erdgas ist Methan.

Die nicht-oxidative Umsetzung von Methan in Gegenwart von Mo oder Zn enthaltenden HZSM-5-Zeolith-Katalysatoren zu Benzol und Wasserstoff gegebenenfalls unter Bildung von Nebenprodukten wie Ethylen, Naphthalin und weiterer, höherer Kohlenwasserstoffe ist länger bekannt (Wang et al., Catalysis Letters 21, 35-41 (1993)) und wird auch dehydrierende Aromatisierung genannt. Nicht-oxidativ bedeutet in diesem Zusammenhang, dass die Umsetzung in Abwesenheit eines Oxidationsmittels, insbesondere in Abwesenheit von Sauerstoff durchgeführt wird.

Liu et al., Applied Catalysis A: General 295, 79-88 (2005) berichten von Selektivitäten zwischen ca. 60 % und 80 % für die nicht-oxidative Umsetzung von Methan zu Benzol und Naphthalin an mit Mo dotierten HZSM-5-Zeolithen, abhängig vom Mo-Gehalt im Katalysator.

Hassan et al., Applied Catalysis A: General 297, 159-164 (2006) erhielten bei der nicht-oxidativen Umsetzung von Methan zu Aromaten an ZSM-5-Zeolithen, die mit Ru und Mo dotiert waren, Methanumsätze von bis zu 6,4 % bei Selektivitäten für Benzol zwischen 62 bis 84 % bei deutlich verlängerten Standzeiten des Katalysators und niedrigen Temperaturen.

Die nicht-oxidative Aromatisierung von Methan und den höheren Alkanen wird durch die Lage des thermodynamischen Gleichgewichts limitiert. Bei der nicht-oxidativen Aromatisierung von Methan werden für jedes gebildete Molekül Benzol 9 Moleküle H₂ frei, so dass diese Reaktion auch zur Gewinnung von reinem H₂ genutzt wird, das beispielsweise in Brennstoffzellen verwendet wird. Die Abtrennung des entstandenen Wasserstoffs von nicht umgesetzten Methan und höheren Alkanen sowie bei der Aromatisierungsreaktion gebildeten Aromaten ist generell schwierig und nur mit hohem apparativem Aufwand durchführbar. Sie kann beispielsweise mittels einer Wasserstoff durchlässigen Membran oder mittels Adsorptionsverfahren erfolgen. Der abgetrennte Wasserstoff kann anschließend weiter verwendet werden.

Bestimmt durch die Lage des thermodynamischen Gleichgewichts sind die Alkanumsätze bei der nicht-oxidativen Aromatisierung niedrig und führen auch bei hohen Selektivitäten der Reaktion zu vergleichsweise geringen Ausbeuten an Aromaten. Der nach Abtrennung der Aromaten aus dem Produktgasstrom erhaltene Gasstrom kann in die Aromatisierungsreaktion zurückgeführt werden, um die Ausbeuten in Bezug auf eingesetzte Alkane zu erhöhen, er enthält jedoch noch den bei der Aromatisierung gebildeten Wasserstoff, der sich ungünstig auf die Lage des thermodynamischen Gleichgewichts auswirkt. Um den ungünstigen Einfluss des Wasserstoffs zu beseitigen, müsste der Wasserstoff vor Rückführung des Gasstroms aus diesem entfernt werden. Wie vorstehend beschrieben ist dies nur mit Hilfe eines vergleichsweise hohen apparativen Aufwands möglich.

Ammoniak stellt ein weiteres wichtiges Zwischenprodukt in der chemischen Industrie dar. Eine Quelle für Ammoniak war das Gaswasser von Kokereien und Gasanstalten. Wesentlich bedeutender ist jedoch die Ammoniaksynthese nach dem Haber-Bosch-Verfahren. Dieses ist seit langem bekannt und auch heutzutage werden ca. 90 % der Weltproduktion an NH₃ nach diesem Verfahren erzeugt. Der für die Synthese benötigte Wasserstoff wird üblicherweise durch Reaktion von Wasserdampf mit Koks oder Kohlenwasserstoffen hergestellt und muss in weiteren Schritten von den dabei entstandenen Kohlenoxiden gereinigt werden.

Ein weiteres Verfahren zur Synthese von Ammoniak wird von Wellenbücher et al. in Zeolites and Related Microporous Materials: State of the Art 1994, Studies in Surface Science and Catalysis, Vol. 84 (1994, 941-948), Elsevier Science beschrieben. Bei Umsetzung einer Mischung aus N₂ und H₂ im Verhältnis von 1 : 3 an Zeolithen des NaY-Typs enthaltend 5 Gew.-% Ru bei Temperaturen von 843 K werden Stickstoffumsätze nahe des thermodynamischen Gleichgewichts erreicht.

Aufgabe der vorliegenden Erfindung ist ein Verfahren zur Herstellung von Aromaten aus C₁- bis C₆-Aliphaten, das zu hohen Ausbeuten an Aromaten führt, wobei möglichst weitgehend auf die mit hohem Aufwand verbundene Abtrennung von Wasserstoff verzichtet wird und die bei der Aromatisierungsreaktion gebildeten Nebenprodukte wirtschaftlich genutzt werden.

Die Aufgabe wird erfindungsgemäß gelöst durch ein Verfahren zur integrierten Herstellung von Aromaten und Ammoniak durch Umsetzung eines mindestens ein C₁-bis C₆-Aliphaten und Stickstoff enthaltenden Gasstroms A in Gegenwart mindestens eines Katalysators, bevorzugt eines Zeolith-Katalysators, wobei in einer Reaktion die C₁- bis C₆-Aliphaten nicht-oxidativ zu Aromaten und der dabei frei werdende Wasserstoff mit Stickstoff in einer weiteren Reaktion zu Ammoniak umgesetzt wird.

Der bei der nicht-oxidativen Aromatisierung in stöchiometrischen Mengen frei werdende Wasserstoff wird dabei ohne Trennschritt zu einem weiteren wirtschaftlich nutzbaren Produkt umgesetzt.

Vorteilhaft an dem erfindungsgemäßen Verfahren ist die gleichzeitige Herstellung zweier bedeutender Grundprodukte der industriellen Chemie. Der neben den Aromaten hergestellte Ammoniak besitzt zudem den Vorteil, dass er aufgrund seines Siedepunktes und seiner sehr guten Löslichkeit in Wasser leicht aus dem Produktstrom abgetrennt werden kann. Des Weiteren wird durch die Ammoniakbildung dem Reaktionsgemisch H₂ entzogen, so dass das Gleichgewicht der Aromatisierungsreaktion auf die Seite der Aromaten verschoben werden sollte. Ein weiterer Vorteil des erfindungsgemäßen Verfahrens liegt in der Kombination einer endothermen (nicht-oxidative dehydrierende Aromatisierung) mit einer exothermen (Ammoniakbildung aus N₂ und H₂) Reaktion, so dass die Energiebilanz des erfindungsgemäßen Verfahrens gegenüber einer reinen, nicht-oxidativen Dehydroaromatisierung deutlich günstiger ist.

Vorteilhaft in Bezug auf die Ammoniaksynthese ist die in situ-Bereitstellung des dafür benötigten Wasserstoffs, der sonst in einem der Ammoniaksynthese vorgeschalteten Verfahren eigens hergestellt werden muss. Zudem entfallen einige sonst notwendige Reinigungsschritte, in denen unerwünschte oder für die weitere Verwendung schädliche, bei der Wasserstoffherstellung entstandene Nebenprodukte abgetrennt werden, wie beispielsweise der Vergleich mit dem Haber-Bosch-Verfahren zeigt.

Erfindungsgemäß enthält der Gasstrom A mindestens einen Aliphaten mit 1 bis 6 Kohlenstoffatomen. Eingesetzt werden können beispielsweise Methan, Ethan, Propan, n-Butan, iso-Butan, n-Pentan, iso-Pentan, n-Hexan, iso-Hexan und 2,3-Dimethylbutan sowie Mischungen davon. Bevorzugt werden Aliphate mit 1 bis 4 Kohlenstoffatomen eingesetzt, also Methan, Ethan, Propan, n-Butan und i-Butan.

Bevorzugt wird als Quelle der C₁- bis C₆-Aliphaten Erdgas eingesetzt. Die typische Zusammensetzung von Erdgas sieht folgendermaßen aus: 75 bis 99 mol-% Methan, 0,01 bis 15 mol% Ethan, 0,01 bis 10 mol-% Propan, bis zu 6 mol-% Butan und höhere Kohlenwasserstoffe, bis zu 30 mol-% Kohlendioxid, bis zu 30 mol-% Schwefelwasserstoff, bis zu 15 mol-% Stickstoff und bis zu 5 mol-% Helium. Das Erdgas kann vor dem Einsatz in dem erfindungsgemäßen Verfahren nach dem Fachmann bekannten Methoden gereinigt und angereichert werden. Zur Reinigung gehört beispielsweise die Entfernung von gegebenenfalls im Erdgas vorhandenen Schwefelwasserstoff oder Kohlendioxid und weiterer, im anschließenden Verfahren unerwünschten Verbindungen.

Das Erdgas kann auch nach dem in der EP 1674555 A der Anmelderin beschriebenen Verfahren vorbehandelt werden. Nach diesem Verfahren wird eine Anreicherung des Methans durch Überleiten des Erdgases über sogenannte MOF's (Engl.: "Metal-Organic-Framework") und reversibler Adsorption der nicht erwünschten Bestandteile wie Kohlendioxid oder höheren Alkane erreicht, wobei die höheren Alkane Ethan, Propan, n-Butan und i-Butan selektiv, d. h. einzeln oder gemeinsam entfernt werden können. Es kann sowohl das nach diesem Verfahren vorbehandelte, mit Methan angereicherte Gas als auch die bei der Vorbehandlung abgetrennten höheren Kohlenwasserstoffen wie Ethan, Propan, n-Butan und i-Butan in dem erfindungsgemäßen Verfahren eingesetzt werden.

In einer weiteren Ausführungsform der vorliegenden Erfindung werden die nach dem vorstehend beschriebenen Verfahren an den MOF's adsorbierten höheren Kohlenwasserstoffe mit mindestens zwei Kohlenstoffatomen nach Desorption in dem erfindungsgemäßen Verfahren eingesetzt.

Besonders bevorzugt wird Erdgas mit hohem Brennwert, das einen hohen Anteil an Kohlenwasserstoffen mit mehr als einem Kohlenstoffatom besitzt, eingesetzt, sogenanntes H-Gas. Bei diesen Kohlenwasserstoffen mit mehr als einem Kohlenstoffatom handelt es sich um Ethan, Propan, n-Butan und i-Butan.

Die im Erdgas gegebenenfalls vorhandenen Verunreinigungen können auch im Gasstrom A enthalten sein.

Die in dem Gasstrom A enthaltenen C₁- bis C₆-Aliphaten können auch aus anderen Quellen stammen. Sie können beispielsweise bei der Erdölraffination angefallen sein. Die C₁- bis C₆-Aliphaten können auch regenerativ gewonnen worden sein (z.B. Biogas) oder synthetisch, beispielsweise durch die Fischer-Tropsch-Synthese, hergestellt worden sein.

Falls als C₁- bis C₆-Aliphaten-Quelle Biogas verwendet wird, kann der Gasstrom A zusätzlich noch Ammoniak, Spuren von niederen Alkoholen und weitere, für Biogas typische Beimischungen enthalten.

Der Gasstrom A enthält N₂, das beispielsweise durch fraktionierte Destillation von verflüssigter Luft gewonnen werden kann. Der Gasstrom A kann deshalb noch Spuren von Edelgasen wie He, Ne oder Ar enthalten.

Generell kann der Gasstrom A alle Verunreinigungen und Beimengungen enthalten, die schon in den Ausgangsstoffen C₁-C₆-Aliphaten und N₂ vorhanden sind.

Das Volumenverhältnis zwischen C₁-C₆-Aliphat zu N₂ im Gasstrom A liegt zwischen 1: 1 und 20 : 1, bevorzugt zwischen 2 : 1 und 15 : 1, besonders bevorzugt zwischen 3 : 1 und 10 : 1.

Die Umsetzung der C₁-C₆-Aliphaten und des N₂ findet unter nicht-oxidativen Bedingungen statt, d.h., weitestgehend in Abwesenheit eines Oxidationsmittels, insbesondere weitestgehend in Abwesenheit von Sauerstoff. Es werden kein oder weitestgehend kein Wasser und ebenso kein oder weitestgehend kein CO bzw. CO₂ aus dem bei der Aromatisierung entstandenem Wasserstoff beziehungsweise den eingesetzten C₁- bis C₆-Aliphaten gebildet.

Erfindungsgemäß findet die Umsetzung des Gasstroms A an mindestens einem Katalysator statt, bevorzugt an mindestens einem Katalysator auf Zeolithbasis.

Beispielsweise können Zeolithe mit Zuordnung zu den folgenden röntgenographischen Strukturtypen eingesetzt werden: ABW, ACO, AEI, AEL, AEN, AET, AFG, AFI, AFN, AFO, AFR, AFS, AFT, AFX, AFY, AHT, ANA, APC, APD, AST, ASV, ATN, ATO, ATS, ATT, ATV, AWO, AWW, BCT, BEA, BEC, BIK, BOG, BPH, BRE, CAN, CAS, SCO, CFI, SGF, CGS, CHA, CHI, CLO, CON, CZP, DAC, DDR, DFO, DFT, DOH, DON, E-AB, EDI, EMT, EON, EPI, ERI, ESV, ETR, EUO, FAU, FER, GIS, GIU, GME, GON, GOO, HEU, IFR, IHW, ISV, ITE, ITH, ITW, IWR, IWW, JBW; KFI, LAU, LEV, LIO, LIT, LOS, LOV, LTA, LTL, LTN, MAR, MAZ, MEI, MEL, MEP, MER, MFI, MFS, MON, MOR, MOZ, MSO, MTF, MTN, MTT, MTW, MWW, NAB, NAT, NES, NON, NPO, NSI, OBW, OFF, OSI, OSO, OWE, PAR, PAU, PHI, PON, RHO, RON, RSN, RTE, RTH, RUT, RWR, RWY, SAO, SAS, SAT, SAV, SBE, SBS, SBT, SFE, SFF, SFG, SFH, SFN, SFO, SGT, SOD, SOS, SSY, STF, STI, STT, TER, THO, TON, TSC, UEI, UFI, UOZ, USI, UTL, VET, VFI, VNI, VSV, WIE, WEN, YUG, ZON oder Mischstrukturen aus zwei oder mehr dieser Typen. Bevorzugt werden Zeolithe des CHA-, des MFI-Typs, des Pentasil-Typs oder des FAU-Typs verwendet, insbesondere ZSM-Zeolithe wie ZSM-5, ZSM-8, ZSM-11, ZSM-23 und ZSM-35, vorzugsweise ZSM-5 oder MCM-Zeolithe wie beispielsweise MCM-22.

Die Zeolithe können neben AI weitere Elemente der 3. Hauptgruppe wie Ga, B oder In enthalten. In einem solchen Fall sind Ga-haltige Zeolithe bevorzugt, die als Framework oder Extraframework vorliegen können. Als Gegenionen für die durch die trivalenten Gerüstkationen erzeugte überschüssige negative Ladung können H⁺, Na⁺, Li⁺, K⁺, Rb⁺, Cs⁺, NH₄⁺, Mg²⁺, Ca²⁺, Sr²⁺ und Ba⁺ im Zeolith enthalten sein.

Die Zeolithe können neben Si weitere Elemente der 4. Haupt- oder Nebengruppe wie Ti, Ge oder Sn enthalten.

Die Zeolithe können mit einem oder mehreren weiteren Metallen aus der Gruppe der Übergangsmetalle dotiert sein. Von diesen werden bevorzugt Mn, Mo, Pd, Pt, Ru, Cu, Co, Fe, Re, W oder Zn eingesetzt, besonders bevorzugt werden mit Ga, Zn, In, Mo, W, Ru oder Pt dotierte Zeolithe eingesetzt.

Die zur Dotierung des Katalysators verwendeten Metalle können nach den dem Fachmann bekannten Verfahren auf den Zeolithen aufgebracht werden. Vor dem Aufbringen der Metalle kann der Zeolith gegebenenfalls in die H-Form überführt werden. Dies kann beispielsweise durch Ionenaustausch mit wässriger NH₄NO₃-Lösung, anschließender Trocknung und gegebenenfalls einer darauf folgenden Calcinierung geschehen.

Die Metalle können nasschemisch in Form wässriger, organischer oder organischwässriger Lösungen ihrer Salze durch Imprägnieren des Zeolithen mit der Salzlösung auf den Zeolithen aufgebracht werden. Als Lösungsmittel kann auch überkritisches CO₂ dienen. Beispielhaft seien als Ru-Salz Ru(NH₃)₆Cl₃ und als Mo-Salz (NH₄)₆MO₇O₂₄ genannt. Falls der Zeolith mit mehr als einem Metall dotiert werden soll, können diese nacheinander als Lösung des jeweiligen Salzes oder zu mehreren gemeinsam in einer Lösung, die die gewünschten Metallsalze enthält, aufgebracht werden. An die nass-chemischen Behandlungen schließt sich das Trocknen im Vakuum bei etwa 100°C und darauf folgend die Calcinierung bei etwa 400 bis 600 °C an.

Die Metalle können auch trocken-chemisch auf den Zeolithen aufgebracht werden, beispielsweise indem eine bei höheren Temperaturen gasförmige Metallverbindung aus der Gasphase auf dem Zeolithen abgeschieden wird. Diese Variante lässt sich zum Beispiel mit den Carbonylverbindungen verschiedener Übergangsmetalle, die im Vakuum sublimierbar sind, durchführen. Auf diese Weise kann beispielsweise Mo als Mo(CO)₆ aus der Gasphase auf dem Zeolithen abgeschieden werden.

Die vorstehend beschriebenen nass-chemischen und trocken-chemischen Verfahren zum Aufbringen der Metalle auf den Zeolithen lassen sich auch kombiniert einsetzen.

Der gegebenenfalls mindestens eines der vorstehend aufgeführten Metalle enthaltende Zeolith kann gemäß den dem Fachmann bekannten Verfahren zu Formkörpern verarbeitet werden. Als formgebende Verfahren sind dabei beispielsweise Versprühen einer den Zeolithen enthaltenden Suspension, Tablettieren, Verpressen im feuchten oder trockenen Zustand, Extrudieren oder weitere, dem Fachmann bekannte Verfahren zu nennen. Mindestens zwei dieser Verfahren können auch kombiniert werden. So ist es beispielsweise möglich, zunächst eine den Zeolithen enthaltende Suspension zu versprühen, beispielsweise in einer Sprühzutrocknung oder einer Sprühgranulation, und das erhaltene Sprühgut, gegebenenfalls nach mindestens einem Waschschritt und/oder mindestens Trocknungs- und/oder Calcinierungsschritt, einer weiteren Verformung wie beispielsweise einer Extrusion zu unterwerfen. Beim Verformen können Hilfsmittel wie beispielsweise Porenbildner, Anteigungsmittel, Bindermaterialien oder auch andere, dem Fachmann bekannte Zusatzstoffe eingesetzt werden. Als mögliche Bindermaterialien sind beispielsweise Metalloxide wie beispielsweise SiO₂, Al₂O₃, TiO₂, ZrO2 oder MgO oder Tone oder Gemische aus zwei oder mehr dieser Verbindungen zu nennen. Weiter ist es möglich, Vorstufen zu diesen Bindermaterialien einzusetzen, wobei im Laufe des Herstellungsverfahrens die genannten Bindermaterialien aus diesen Vorstufen gebildet werden. Beispiele für solche Bindermaterialvorstufen sind etwa Tetraalkoxysilane, Tetraalkoxytitanate, Tetraalkoxyzirkonate oder Mischungen aus zwei oder mehr dieser Vorstufen. Als mögliche Anteigungsmittel sind solche Verbindungen zu nennen, die zur Verbesserung der Misch-, Knet und Fließeigenschaften führen. Vorzugsweise sind dies im Rahmen der vorliegenden Erfindung organische, insbesondere hydrophile Polymere wie beispielsweise Cellulose, Cellulosederivate wie beispielsweise Methylcellulose, Stärke wie beispielsweise Kartoffelstärke, Tapetenkleister, Acrylate, Polyacrylate, Polymethacrylate, Polyvinylalkohole, Polyvinylpyrrolidon, Polyisobuten, Polytetrahydrofuran, Polyglykolether, Fettsäureverbindungen, Wachsemulsionen, Wasser oder Mischungen aus zwei oder mehr dieser Verbindungen. Als Porenbildner sind im Rahmen der vorliegenden Erfindung beispielsweise in Wasser oder wässrigen Lösungsmittelgemischen dispergier-, suspendier- oder emulgierbare Verbindungen wie beispielsweise Polyalkylenoxide wie Polyethylenoxide, Polystyrol, Polyacrylate, Polymethacrylate, Polyolefine, Polyamide, Polyester, Kohlenhydrate, Cellulose, Cellulosederivate wie beispielsweise Methylcellulose, Zucker, Naturfasern, Pulp, Graphit oder Mischungen aus zwei oder mehr dieser Verbindungen zu nennen. Porenbildner und/oder Anteigungsmittel werden nach der Verformung bevorzugt durch mindestens einen geeigneten Trocknungs- und/oder Calcinierungsschritt aus dem erhaltenen Formkörper entfernt.

Die Geometrien der erfindungsgemäß erhaltenen Katalysatoren kann beispielsweise kugelförmig (hohl oder voll), zylindrisch (hohl oder voll), ring-, sattel-, stern-, bienenwaben- oder tablettenförmig sein. Weiterhin kommen Extrudate zum Beispiel in Strang-, Trilobe-, Quatrolobe-, Stern- oder Hohlzylinderform in Frage. Der Katalysator kann auch pulverförmig vorliegen. Ebenso sind Voll- oder Hohl-Mikrokugeln denkbar, wie sie beispielsweise aus oben genannten Sprühverfahren erhalten werden können.

Gemäß einer weiteren Ausführungsform der Erfindung können auch zunächst die Formkörper aus dem Zeolithen hergestellt werden und die gewünschten Metalle anschließend nach den vorstehend beschriebenen Verfahren auf die Formkörper aufgebracht werden.

In dem erfindungsgemäßen Verfahren kann ein Katalysator eingesetzt werden, es können jedoch auch zwei oder mehr unterschiedliche Katalysatoren eingesetzt werden. Die unterschiedlichen Katalysatoren können sich nebeneinander, miteinander vermischt oder in Schichten in einer Reaktionszone oder in zwei oder mehr aufeinander folgenden Reaktionszonen befinden. Die Reaktionszonen können in einem Reaktor oder in mehreren Reaktoren angeordnet sein.

Gemäß einer bevorzugten Ausführungsform der Erfindung enthält der Katalysator zwischen 0,2 und 10 Gew.-% Mo, bevorzugt zwischen 0,4 und 8 Gew.-%, besonders bevorzugt zwischen 0,5 und 6 Gew.-% Mo und zwischen 0,02 und 6 Gew.-% Ru, bevorzugt zwischen 0,04 und 5, besonders bevorzugt zwischen 0,05 und 4 Ru.

Im Verlauf der Reaktion kann es zu einem Verlust an den zur Dotierung des Katalysators verwendeten Metallen kommen, so dass deren Konzentration im bzw. auf dem Katalysator nicht mehr der gewünschten Konzentration entspricht. Um diesen Verlust an zur Dotierung des Katalysators verwendeten Metallen auszugleichen, können die Metalle in Form ihrer Carbonyle erneut auf den Katalysator aufgebracht werden.

Erfindungsgemäß kann die Umsetzung des C₁- bis C₆-Alkan und N₂ enthaltenden Gasstroms A grundsätzlich in allen aus dem Stand der Technik bekannten Reaktortypen, die für Feststoff-katalysierte Gasphasenreaktionen geeignet sind, durchgeführt werden.

Geeignete Reaktorformen für den Einsatz in dem erfindungsgemäßen Verfahren sind beispielsweise Rohr- oder Rohrbündelreaktoren, wobei die Katalysatoren weiter bevorzugt als Festbettkatalysatoren eingesetzt werden. Dabei befindet sich der Katalysator als Festbett in einem Reaktionsrohr oder in einem Bündel von Reaktionsrohren. Sowohl Rieselbettfahrweise als auch Sumpffahrweise sind hierbei als mögliche Verfahrensweisen weiter zu nennen. Die Rohrinnendurchmesser können über einen weiten Bereich variieren, d.h., sie können in einem Bereich beginnend bei Mikrometern bis zu einer Größenordnung von Metern liegen. Übliche bevorzugte großtechnische Reaktionsrohr-Innendurchmesser betragen etwa 2 bis 5 cm. Es können jedoch auch kleinere Reaktionsrohr-Innendurchmesser verwendet werden, beispielsweise in Mini- und Mikroreaktoren. In einer weiteren Ausführungsform liegen die Rohrinnendurchmesser zwischen 0,5 und 20 mm.

Ein typischer Rohrbündelreaktor umfasst ca. 2 bis 30.000 Reaktionsrohre.

Die integrierte Herstellung von Ammoniak und Aromaten kann auch im Wirbelbett- oder Wanderbettreaktor durchgeführt werden. Ebenso geeignet ist ein Hordenreaktor. Dieser enthält ein oder mehrere aufeinander folgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einem Hordenreaktor mit nur einer Horde. Die dehydrierende Aromatisierung in Schritt 3) wird bevorzugt in einem Rohrbündel- oder Wirbelschichtreaktor durchgeführt.

Erfindungsgemäß kann der Reaktor ein Reaktorbett enthalten, es kann aber auch zweckmäßig sein, mehrere Reaktorbetten nebeneinander zu betreiben, von denen sich eines oder mehrere in der Regel im Zustand der Regenerierung oder Reaktivierung befinden.

Vor der Reaktion kann der Katalysator durch Erhitzen auf Temperaturen zwischen 350 bis 450 °C in Inertgasatmosphäre, beispielsweise unter He oder Ar, aktiviert werden.

Die Aktivierung kann auch mit einem Methan-haltigen Gaststrom oder einem C₂-C₄-Alkan, beispielsweise Ethan, Propan, Butan oder einem Gemisch aus diesen, erfolgen, vorzugsweise wird Butan verwendet. Die Aktivierung wird bei einer Temperatur von 350 bis 650°C, vorzugsweise bei 400 bis 550°C, und einem Druck von 0,5 bis 5 bar, vorzugsweise bei 0,5 bis 2 bar, durchgeführt. Üblicherweise liegt die GHSV (Gas Hourly Space Velocity) bei der Aktivierung bei 100 bis 4000 h⁻¹, vorzugsweise bei 500 bis 2000 h⁻¹.

Es ist auch möglich, eine Aktivierung durch die im Gasstrom A enthaltenen Kohlenwasserstoffe mit mindestens 2 C-Atomen durchzuführen. Die Aktivierung wird bei einer Temperatur von 250 bis 650°C, vorzugsweise bei 350 bis 550°C, und einem Druck von 0,5 bis 5 bar, vorzugsweise bei 0,5 bis 2 bar, durchgeführt. Üblicherweise liegt die GHSV (Gas Hourly Space Velocity) bei der Aktivierung bei 100 bis 4000 h⁻¹, vorzugsweise bei 500 bis 2000 h⁻¹.

In einer weiteren Ausgestaltungsform ist es auch möglich, zusätzlich noch Wasserstoff beizufügen.

Selbstverständlich kann der eingesetzte Katalysator bei nachlassender Aktivität nach üblichen, dem Fachmann bekannten Methoden regeneriert werden. Hier sei insbesondere die Behandlung mit einem sauerstoffhaltigen Gemisch wie Luft, angereicherter Luft oder Reinsauerstoff aufgeführt, bei der das sauerstoffhaltige Gemisch anstelle des Gasstroms A über den Katalysator geleitet wird. Es ist beispielsweise aber auch möglich, den Katalysator mit Wasserstoff zu regenerieren. Dies kann erfolgen, indem man dem Gasstrom A beispielsweise einen Wasserstoffstrom zusetzt. Das Verhältnis von Wasserstoffstrom zu Gasstrom A liegt üblicherweise im Bereich von 1:1000 bis 2:1, bevorzugt 1:500 bis 1:5.

Erfindungsgemäß wird das Verfahren zur integrierten Herstellung von Aromaten und Ammoniak an den oben genannten Katalysatoren bei Temperaturen von 400 bis 1000°C, bevorzugt von 450 bis 900°C, besonders bevorzugt von 500 bis 800°C, insbesondere von 550 bis 750 °C, bei einem Druck von 0.5 bis 100 bar, bevorzugt bei 1 bis 50 bar, besonders bevorzugt bei 1 bis 30 bar, insbesondere 1 bis 10 bar, durchgeführt. Üblicherweise wird die Umsetzung bei einer GHSV (Gas Hourly Space Velocity) von 100 bis 10 000 h⁻¹, vorzugsweise von 200 bis 6000 h⁻¹ durchgeführt.

Der nach der erfindungsgemäßen Umsetzung erhaltene Produktgasstrom enthält die bei der Aromatisierungsreaktion gebildeten Aromaten wie Benzol, Toluol, Xylol und Naphthalin, bei der Aromatisierungsreaktion möglicherweise auftretende Nebenprodukte wie Ethylen und Acetylen, sowie Ammoniak und aus der Aromatisierungsreaktion stammenden, nicht umgesetzten Wasserstoff. Weitere Bestandteile des Produktgasstroms sind nicht umgesetzte Ausgangsstoffe, d.h. C₁-C₆-Aliphaten und Stickstoff sowie die in ihnen enthaltenen Verunreinigungen.

Gemäß einer Ausführungsform der Erfindung werden aus dem nach der Umsetzung erhaltenen Gasstrom zunächst die Aromaten abgetrennt. Die Abtrennung der Aromaten erfolgt durch Kondensation oder auch fraktionierte Kondensation. So kann das Gemisch auf -30 °C bis 80°C, vorzugsweise auf 0 °C bis 70 °C, besonders bevorzugt auf 30 °C bis 60 °C abgekühlt werden. Hierbei kondensieren die aromatischen Kohlenwasserstoffe, während die nicht-umgesetzen C₁-C₆-Aliphaten, der Stickstoff, der gebildete Ammoniak sowie weitere, bei der Umsetzung gebildete Nebenprodukte sowie gegebenenfalls bereits im Gasstrom A enthaltene Verunreinigungen gasförmig vorliegen und somit nach üblichen Methoden abgetrennt werden können. Der Produktgasstrom kann vor oder nach dem Abkühlen auch verdichtet und gegebenenfalls weiter gekühlt werden. Eine Verdichtung erfolgt vorzugsweise auf einem Druckniveau von 1 bis 100 bar, bevorzugt 1 bis 75 bar und weiter bevorzugt 5 bis 15 bar. Um eine weitgehende Kondensation einer bestimmten Verbindung zu erreichen, wird eine entsprechend angemessene Temperatur eingestellt.

Aus dem von Aromaten befreiten Produktgasstrom kann durch weiteres Abkühlen der darin enthaltene Ammoniak kondensiert und abgetrennt werden. Unter Atmosphärendruck wird dies bevorzugt bei Temperaturen unterhalb von -33 °C durchgeführt. Alternativ kann der Ammoniak auch mittels Durchleiten des Gasstroms durch Wasser abgetrennt werden.

Der Ammoniak kann auch durch reversible Adsorption und Desorption (thermisch oder Druckwechsel-Adsorption) aus dem von Aromaten befreiten Produktgasstrom entfernt werden.

Gemäß einer weiteren Ausführungsform der Erfindung kann der nicht bei der Ammoniak-Synthese verbrauchte Wasserstoff durch übliche, dem Fachmann bekannte Verfahren aus dem Gasstrom entfernt werden, beispielsweise indem der Gasstrom entlang einer selektiv für molekularen Wasserstoff durchlässigen Membran geführt wird, oder indem die übrigen im Gasstrom enthaltenen Komponenten reversibel adsorbiert werden (thermisch oder Druckwechsel-Adsorption) und anschließend wieder desorbiert werden.

Der von Aromaten, Ammoniak und gegebenenfalls Wasserstoff befreite Gasstrom kann teilweise oder vollständig in die Reaktionszone zurückgeführt werden. Gegebenenfalls können aus dem Gasstrom auch die C₁-bis C₆-Aliphaten abgetrennt und in die Reaktionszone zurückgeführt werden.

Im Folgenden wird die Erfindung anhand von Beispielen erläutert.

### Beispiele

Die Umsetzung in den Beispielen 2, 3 und 4 werden in einer kommerziell verfügbaren TPR-TPO-TPD-Apparatur (AutoChemll2920, Fa. Micromeritics mit Massenspektrometer OmniStar und QMS200, Fa. Pfeiffer Vacuum) durchgeführt. Die Apparatur enthält einen Quarzglasreaktor mit einer Länge von 205 mm und einem Innendurchmesser von 9 mm.

### Beispiel 1 Herstellung des Katalysators, erfindungsgemäß

500 g eines Zeolithpulvers mit MFI-Struktur (PZ 2/25; Fa. Zeochem, Uetikon, Schweiz) werden in einem Glas-Dreihalskolben mit 5 kg einer Lösung aus 500 g Ammoniumnitrat in 4500 g deionisiertem Wasser für 2 Stunden bei 80 °C unter Rühren behandelt. Danach wird der Feststoff abfiltriert, mit Wasser neutralgewaschen und über Nacht bei 120 °C in Luft getrocknet. Die Auswaage an Feststoff beträgt 592 g.

Von diesem Feststoff werden in einem kleineren Rührkolben 20 g mit einer Lösung aus 0,092 g Hexaminruthenium-III-chlorid in 1000 g deionisiertem Wasser für die Dauer von fünfzig Stunden bei Raumtemperatur gerührt. Die entstandene Suspension wird abfiltriert, neutralgewaschen und wie vorgängig beschrieben getrocknet. Die Auswaage beträgt 19,5 g.

In einem Drehrohr werden 17 g des Feststoffes aus der Rutheniumbehandlung 2 Stunden lang bei 400 °C im Heliumstrom behandelt (Aufheizrate: 4°C/min). Nach Abkühlen wird das graue Pulver in einer Stickstoffatmosphäre in ein Schlenkrohr überführt, die Auswaage beträgt 14 g.

In einem zweiten Schlenkrohr werden 2,5 g Molybdänhexacarbonyl vorgelegt und zur Reinigung insgesamt 3 x im Vakuum bei 150 °C umsublimiert, wobei sich das gereinigte Molybdänhexacarbonyl an den Wänden im oberen Bereich des Schlenkrohres abscheidet.

In das Schlenkrohr mit dem vorgereinigten Molybdänhexacarbonyl werden ca. 2 cm oberhalb der Schüttung des Carbonylpulvers eine Portion Quarzglaswolle und auf der Wolle deponiert das rutheniumbehandelte Zeolithpulver gegeben. Anschließend wird bei Raumtemperatur eine Stunde lang bis zu einem Druck von 8 bis 12 mbar evakuiert, anschließend wird das Gefäß in ein Ölbad gesetzt und auf 130 °C erwärmt. Nachdem das Molybdänhexacarbonyl am Boden des Gefäßes vollständig verdampft ist, wird das Glasgefäß abgekühlt und für weitere 24 Stunden bei 80 °C gehalten. Danach wird auf Raumtemperatur abgekühlt und mit Inertgas (Stickstoff) belüftet.

### Beispiel 2 Erzeugung von Ammoniak aus Stickstoff und Wasserstoff, nicht erfindungsgemäß

Die Apparatur inklusive Reaktor werden zunächst drucklos mit einem Fluss von 30 ml/min Helium (Fa. Praxair, Reinheit 6.0) gespült, danach mit einer Heizrate von 10°C/min auf 400 °C gefahren und für 120 Minuten bei dieser Temperatur gehalten. Anschließend wird der Reaktor unter einem Fluss von 25 ml/min Helium und einer Heizrate von 10°C/min auf 450°C aufgeheizt und für 30 Minuten bei diesen Bedingungen gehalten. In dem Reaktor sind 51 mg Katalysator aus Beispiel 1 als Mikrofestbett mit Glaswolle als Katalysatorstuhl fixiert.

Der Heliumfluss wird auf einen Fluss von 25 ml/min einer Mischung aus 25 Vol.-% Stickstoff (Fa. Air Liquide, Reinheit 4.6) und 75 Vol.-% Wasserstoff (Fa. Air Liquide, Reinheit 5.0) umgestellt. Mit einer Rate von 2°C/min wird auf 550 °C aufgeheizt und für eine Stunde gehalten, danach mit 2°C/min weiter auf 650°C aufgeheizt und 60 Minuten lang gehalten.

Das Gas im Ausgang des Reaktors wird in einer Kühlfalle bei -196°C gesammelt. Der in der Kühlfalle aufgefangene Austrag wird von zwei unterschiedlichen Testpersonen bewertet, die übereinstimmend stechenden Ammoniakgeruch feststellen.

### Beispiel 3 Erzeugung von Aromaten und Ammoniak aus Methan und Stickstoff, erfindungsgemäß

Die Apparatur inklusive des Reaktors werden zunächst drucklos mit einem Fluss von 30 ml/min Helium (Fa. Praxair, Reinheit 6.0) gespült, anschließend mit einer Heizrate von 10°C/min auf 400°C aufgeheizt, unter diesen Bedingungen 120 Minuten lang gehalten, daraufhin bei einem Heliumfluss von 25 ml/min und einer Heizrate von 10°C/min auf eine Reaktortemperatur von 450°C aufgeheizt und 30 Minuten lang gehalten. In dem Quarzglasreaktor sind 198 mg Katalysator (aus Beispiel 1) als Mikrofestbett mit Glaswolle als Katalysatorstuhl fixiert.

Die Reaktortemperatur wird mit einer Heizrate von 10°C/min von 450°C auf 500 °C erhöht und nach einer Haltezeit von ca. 15 Minuten weiter mit einer Heizrate von 30°C/min auf maximal 650°C angehoben und bei dieser Temperatur für die Dauer von 30 Minuten gehalten. Bei dieser Temperatur wird auf einen Fluss von 25 ml/min einer Gasmischung aus 58 Vol.-% Methan (Fa. Air Liquide, Reinheit 2.5) und 42 Vol.-% Stickstoff umgeschaltet. Das Gas am Ausgang des Reaktors wird über eine Zeit von mehreren Stunden in einer Kühlfalle bei -196°C gesammelt, mit 2,6 g Ethanol (p.a.) aufgenommen und analysiert. Der Gehalt an Ammoniak in dem gesammelten Austrag wird ionenchromatographisch bestimmt und beträgt 10 mg/kg. Der gaschromatographisch bestimmte Benzolgehalt liegt bei 30 mg/kg. Der ausgebaute Katalysator ist tiefschwarz gefärbt und hat ein Gewicht von 259 mg.

## Patentansprüche

1. Verfahren zur integrierten Herstellung von Aromaten und Ammoniak durch Umsetzung eines mindestens einen C₁- bis C₆-Aliphaten und Stickstoff enthaltenden Gasstroms A in Gegenwart mindestens eines Katalysators, wobei in einer Reaktion die C₁- bis C₆-Aliphaten nicht-oxidativ zu Aromaten und der dabei frei werdende Wasserstoff mit Stickstoff in einer weiteren Reaktion zu Ammoniak umgesetzt wird.

2. Verfahren gemäß Anspruch 1, **dadurch gekennzeichnet, dass** der oder die Katalysatoren Zeolith enthält.

3. Verfahren gemäß Anspruch 1 oder 2, **dadurch gekennzeichnet, dass** der Katalysator mit einem oder mehreren Metallen aus der Gruppe der Übergangsmetalle dotiert sein.

4. Verfahren gemäß einem der Ansprüche 1 bis 3, **dadurch gekennzeichnet, dass** der oder die Katalysatoren mit einem oder mehreren Übergangsmetallen ausgewählt aus der Gruppe Mn, Mo, Pd, Pt, Ru, Cu, Co, Fe, Re, W und Zn dotiert ist.

5. Verfahren gemäß einem der Ansprüche 1 bis 4, **dadurch gekennzeichnet, dass** der Katalysator von 0,2 bis 10 Gew.-% Mo und von 0,02 bis 6 Gew.-% Ru enthält.

6. Verfahren gemäß einem der Ansprüche 1 bis 5, **dadurch gekennzeichnet, dass** der oder die Katalysatoren Zeolith vom MFI-Typ enthält.

7. Verfahren gemäß einem der Ansprüche 1 bis 6, **dadurch gekennzeichnet, dass** als C₁- bis C₆-Aliphat Erdgas eingesetzt wird.

8. Verfahren gemäß einem der Ansprüche 1 bis 7, **dadurch gekennzeichnet, dass** das Verhältnis von C₁- bis C₆-Aliphat zu N₂ bezogen auf die Volumina im Gasstrom A zwischen 1 : 1 und 20 : 1 beträgt.

9. Verfahren gemäß einem der Ansprüche 1 bis 8, **dadurch gekennzeichnet, dass** die Umsetzung bei Temperaturen zwischen 400 °C und 1000 °C und Drücken zwischen 0,5 bar und 100 bar durchgeführt wird.

10. Verfahren gemäß einem der Ansprüche 1 bis 10, **dadurch gekennzeichnet, dass** die Umsetzung mit Gasgeschwindigkeiten von 100 bis 10000 h⁻¹ durchgeführt wird.

11. Verfahren gemäß einem der Ansprüche 1 bis 11, **dadurch gekennzeichnet, dass** die Umsetzung in einem Festbett- oder Wirbelschichtreaktor durchgeführt wird.

## Claims

1. A process for the integrated preparation of aromatics and ammonia by reaction of a gas stream A comprising at least one C₁-C₆-aliphatic and nitrogen in the presence of at least one catalyst, wherein the C₁-C₆-aliphatics are converted nonoxidatively into aromatics in one reaction and the hydrogen liberated in this reaction is reacted with nitrogen to form ammonia in a further reaction.

2. The process according to claim 1, wherein the catalyst or catalysts comprises/comprise zeolite.

3. The process according to claim 1 or 2, wherein the catalyst is doped with one or more transition metals.

4. The process according to any of claims 1 to 3, wherein the catalyst or catalysts is/are doped with one or more transition metals selected from the group consisting of Mn, Mo, Pd, Pt, Ru, Cu, Co, Fe, Re, W and Zn.

5. The process according to any of claims 1 to 4, wherein the catalyst comprises from 0.2 to 10% by weight of Mo and from 0.02 to 6% by weight of Ru.

6. The process according to any of claims 1 to 5, wherein the catalyst or catalysts comprises/comprise zeolite of the MFI type.

7. The process according to any of claims 1 to 6, wherein natural gas is used as C₁-C₆ aliphatic.

8. The process according to any of claims 1 to 7, wherein the ratio of C₁-C₆ aliphatic to N₂ based on the volumes in the gas stream A is from 1 : 1 to 20 : 1.

9. The process according to any of claims 1 to 8, wherein the reaction is carried out at temperatures of from 400°C to 1000°C and pressures of from 0.5 bar to 100 bar.

10. The process according to any of claims 1 to 9, wherein the reaction is carried out at gas space velocities of from 100 to 10 000 h⁻¹.

11. The process according to any of claims 1 to 10, wherein the reaction is carried out in a fixed-bed or fluidized-bed reactor.

## Revendications

1. Procédé de fabrication intégrée de composés aromatiques et d'ammoniac par mise en réaction d'un courant gazeux A contenant au moins un composé aliphatique en C₁ à C₆ et de l'azote en présence d'au moins un catalyseur, les composés aliphatiques en C₁ à C₆ réagissant de manière non oxydative pour former des composés aromatiques lors d'une réaction et l'hydrogène ainsi libéré réagissant avec l'azote lors d'une réaction supplémentaire pour former de l'ammoniac.

2. Procédé selon la revendication 1, **caractérisé en ce que** le ou les catalyseurs contiennent une zéolithe.

3. Procédé selon la revendication 1 ou 2, **caractérisé en ce que** le catalyseur est dopé avec un ou plusieurs métaux du groupe des métaux de transition.

4. Procédé selon l'une quelconque des revendications 1 à 3, **caractérisé en ce que** le ou les catalyseurs sont dopés avec un ou plusieurs métaux de transition choisis dans le groupe Mn, Mo, Pd, Pt, Ru, Cu, Co, Fe, Re, W et Zn.

5. Procédé selon l'une quelconque des revendications 1 à 4, **caractérisé en ce que** le catalyseur contient de 0,2 à 10 % en poids de Mo et de 0,02 à 6 % en poids de Ru.

6. Procédé selon l'une quelconque des revendications 1 à 5, **caractérisé en ce que** le ou les catalyseurs contiennent une zéolithe de type MFI.

7. Procédé selon l'une quelconque des revendications 1 à 6, **caractérisé en ce que** du gaz naturel est utilisé en tant que composé aliphatique en C₁ à C₆.

8. Procédé selon l'une quelconque des revendications 1 à 7, **caractérisé en ce que** le rapport entre le composé aliphatique en C₁ à C₆ et N₂ par rapport aux volumes dans le courant gazeux A est compris entre 1:1 et 20:1.

9. Procédé selon l'une quelconque des revendications 1 à 8, **caractérisé en ce que** la réaction est réalisée à des températures comprises entre 400 °C et 1 000 °C et à des pressions comprises entre 0,5 bar et 100 bar.

10. Procédé selon l'une quelconque des revendications 1 à 9, **caractérisé en ce que** la réaction est réalisée à des vitesses de gaz de 100 à 10 000 h⁻¹.

11. Procédé selon l'une quelconque des revendications 1 à 10, **caractérisé en ce que** la réaction est réalisée dans un réacteur à lit fixe ou à lit fluidisé.
